# EUROPÄISCHE PATENTSCHRIFT

(11) **EP 4 722 379 B1**
(45) Veröffentlichungstag und Bekanntmachung des Hinweises auf die Patenterteilung: **21.10.2026**
(21) Anmeldenummer: 24204249.7
(22) Anmeldetag: 02.10.2024
(51) Int. Cl.: C12Q 1/34, G01N 33/58, G01N 33/94, G01N 33/68

(54) **VERFAHREN ZUM BESTIMMEN DES GEHALTS WENIGSTENS EINES ANALYTEN IN EINER PROBE UND ANALYSE-KIT ZUR VERWENDUNG BEI DIESEM VERFAHREN**
METHOD FOR DETERMINING THE CONTENT OF AT LEAST ONE ANALYTE IN A SAMPLE AND ANALYSIS KIT FOR USE IN SAID METHOD
PROCÉDÉ DE DÉTERMINATION DE LA TENEUR D'AU MOINS UN ANALYTE DANS UN ÉCHANTILLON ET KIT D'ANALYSE DESTINÉ À ÊTRE UTILISÉ DANS CE PROCÉDÉ

(43) Veröffentlichungstag der Anmeldung: 08.04.2026
(73) Patentinhaber: Chromsystems Instruments & Chemicals GmbH, 82166 Gräfelfing (DE)
(72) Erfinder: SCHWARZFISCHER, Philipp, Dr., 82166 Gräfelfing (DE); BÖTTCHER, Michael, Dr., 22395 Hamburg (DE)
(74) Vertreter: Zenz Patentanwälte Partnerschaft mbB

(56) Entgegenhaltungen:
- EP-A1- 2 671 642
- US-A1- 2016 231 341
- US-A1- 2018 067 116
- JAGANI RAVIKUMAR ET AL: "Validated single urinary assay designed for exposomic multi-class biomarkers of common environmental exposures", ANALYTICAL AND BIOANALYTICAL CHEMISTRY, SPRINGER BERLIN HEIDELBERG, BERLIN/HEIDELBERG, vol. 414, no. 19, 27 June 2022 (2022-06-27), pages 5943 - 5966, XP037906017, ISSN: 1618-2642, [retrieved on 20220627], DOI: 10.1007/S00216-022-04159-4
- RAPS MIRIAM ET AL: "Quantitative analysis of 34 sex (pro)hormones, conjugates and bioactive oxidation products thereof in human plasma by GC- and LC-MS/MS and systematic investigation of overestimations of analyte concentrations not accounted for by method validation", STEROIDS, ELSEVIER SCIENCE PUBLISHERS, NEW YORK, NY, US, vol. 208, 18 May 2024 (2024-05-18), XP087556645, ISSN: 0039-128X, [retrieved on 20240518], DOI: 10.1016/J.STEROIDS.2024.109441

## Beschreibung

Die Erfindung betrifft ein Verfahren zum Bestimmen des Gehalts wenigstens eines Analyten in einer Probe, wobei der wenigstens eine Analyt zumindest teilweise als Glucuronid vorliegt, wobei das wenigstens eine Glucuronid durch Hydrolyse unter Freisetzung des wenigstens einen Analyten aufgespalten wird, wobei der Probe für jeden Analyten eine vorgegebene Menge eines Isotopologs des Analyten als interner Standard zugegeben wird, wobei die Probe nach der Hydrolyse einer quantitativen Analyse zugeführt wird, bei der für jeden Analyten ein Verhältnis des Gehalts des Analyten zu dem Gehalt des zugehörigen Isotopologs bestimmt wird.

Ferner betrifft die Erfindung ein Analyse-Kit zur Verwendung bei einem Verfahren zum Bestimmen des Gehalts wenigstens eines Analyten in einer Probe, in der der wenigstens eine Analyt zumindest teilweise als Glucuronid vorliegt, wobei das Verfahren eine Hydrolyse des wenigstens einen Glucuronids und eine nachfolgende quantitative Analyse des wenigstens einen Analyten umfasst.

Ein Verfahren und ein Analyse-Kit der eingangs genannten Art sind aus der Broschüre "MassTox^{®} - Drogenanalytik im Urin mit LC-MS/MS" der Chromsystems Instruments & Chemicals GmbH bekannt (die Broschüre ist bestellbar unter https://chromsystems.com/de/produkte/drogenanalytik.html). Mit dem bekannten Verfahren können mittels Flüssigchromatographie (LC) mit nachfolgender Massenspektrometrie (MS) in einem Durchlauf das Vorhandensein (Target Screening) und der Gehalt von mehr als 100 Drogen und Metaboliten in Urinproben bestimmt werden. Dabei werden der Probe vor der Analyse mittels HPLC-MS/MS vorgegebene Mengen sogenannter interner Standards zugegeben werden, wobei für jede zu analysierende Droge (jeden Analyten) ein geeigneter interner Standard zugegeben wird. Dabei kann ein interner Standard auch für mehrere ähnliche Drogen verwendet werden. Bei dem bekannten Verfahren gibt es für 98 Analyten eigene isotopenmarkierte interne Standards.

Bei einem internen Standard handelt es sich um einen Stoff, der gleichzeitig mit der zu analysierenden Droge in derselben Probe analysiert werden kann und der sich bei der Analyse wie die Droge selbst oder sehr ähnlich verhält, aber dabei, insbesondere bei der Massenspektrometrie, noch von der Droge unterscheidbar ist. Der interne Standard ist so beschaffen, dass sich alle präanalytischen und analytischen Schritte, die nach der Zugabe des internen Standards zu der Probe ausgeführt werden, auf die Droge und den zugehörigen internen Standard gleich oder sehr ähnlich auswirken. Als interner Standard einer Droge wird hierbei vorzugsweise ein Isotopolog der Droge, d. h. eine chemische Verbindung verwendet, die sich von der Droge durch eine Isotopen-Zusammensetzung unterscheidet. Bei Drogen, bei denen es sich um Kohlenwasserstoffverbindungen handelt, sind vorzugsweise mehrere Wasserstoffatome durch Deuteriumatome ersetzt; die Verbindung ist "deuteriert". Alternativ oder zusätzlich können Kohlenstoffatome durch stabile Isotope des Kohlenstoffs und/oder Stickstoffatome durch stabile Isotope des Stickstoffs ersetzt sein. Es ist anzumerken, dass Isotopologe der Droge auch natürlich vorkommen können. Die als interne Standards verwendeten Isotopologe sollten daher solche sein, die nicht oder nur sehr selten natürlich auftreten. Beispielsweise werden Isotopologe eingesetzt, bei denen mindestens drei der schwereren Atome (Kohlenstoff, Stickstoff, Sauerstoff) durch stabile Isotope (z.B. ¹³C, ¹⁵N, ¹⁷O, ¹⁸O) ersetzt werden.

Der Gehalt (d. h. der Massen-, Stoffmengen- oder Volumenanteil oder die Massen-, Stoffmengen- oder Volumenkonzentration) des internen Standards in der Probe bei dessen Zugabe ist bekannt. Der anschließend bei der Analyse bestimmte Gehalt kann somit mit dem bekannten Gehalt verglichen werden. Hat sich der bei der Analyse bestimmte Gehalt gegenüber dem bekannten Gehalt verändert, so kann mit guter Näherung davon ausgegangen werden, dass sich der Gehalt des Analyten (der Droge) in gleicher Relation verändert hat. Deshalb erlaubt die Bestimmung des Verhältnisses des Gehalts der Droge zu dem Gehalt des internen Standards auf der Basis des bekannten Gehalts des internen Standards bei dessen Zugabe zu der Probe eine Bestimmung des Gehalts der Droge in der Probe vor der Analyse. Der eigentlichen Analyse geht üblicherweise eine Kalibrierung voraus, bei der eine Kalibrierungslösung verwendet wird, die sowohl eine oder vorzugsweise mehrere Drogen als auch deren interne Standards jeweils in bekannter Konzentration enthält.

Bei der Untersuchung von Urinproben ist zusätzlich zu berücksichtigen, dass viele Drogen, insbesondere solche Drogen, die als unpolare Stoffe hydrophob sind, überwiegend nicht in ihrer reinen Form, sondern vielmehr glucuronidiert vorliegen. Glucuronidierung bezeichnet den Vorgang der Ausscheidung unpolarer Stoffe über Leber und Niere durch Bindung an Glucuronsäure. Die Drogen werden in der Leber glucuronidiert und die so gebildeten Glucuronide, die im Vergleich zu der ursprünglichen Droge wesentlich hydrophiler sind, können besser über die Niere ausgeschieden werden.

Damit der Gehalt der Droge in der Urinprobe unter der oben beschriebenen Zugabe eines Isotopologs der Droge bestimmt werden kann, ist es erforderlich, die Glucuronide der Droge zu hydrolysieren, d. h. in Glucuronsäure und die Droge aufzuspalten. Derjenige Anteil der Droge, der in Form eines Glucuronids vorliegt, kann bei der Analyse nicht erfasst werden. Bei dem bekannten Verfahren werden ausschließlich nicht glucuronidierte (=freie) Drogen erfasst. Deshalb werden bei dem genannten bekannten Verfahren der Probe vor der Analyse Enzyme, genauer gesagt β-Glucuronidasen zugegeben, die die Glucuronid-Metaboliten zurück zur nativen Droge hydrolysieren, wie z.B in Dokumenten US2016231341 und US2018067116 beschrieben.

Um den Drogengehalt in der ursprünglichen Probe korrekt zu bestimmen, ist es erwünscht, dass sämtliche Glucuronide der Droge oder zumindest der weitaus überwiegende Teil durch die β-Glucuronidase hydrolysieren worden sind, bevor die Probe der quantitativen Analyse zugeführt wird. Allerdings ist die Aktivität von Enzymen, wie der β-Glucuronidase, unter anderem von der Affinität zu einem bestimmten Substrat (hier dem Glucuronid der Droge), der Konzentration des Substrats, dem pH-Wert der Lösung, der Temperatur und dem Vorhandensein von kompetitiven oder allosterischen Inhibitoren sowie von der Dauer der Hydrolyse abhängig. Diese komplexen Abhängigkeiten, welche sich wechselseitig beeinflussen, führen dazu, dass es nicht möglich ist vorherzusagen, ob und in welchem Ausmaß in einer bestimmten Probe nach Zugabe von β-Glucuronidase eine Hydrolyse stattgefunden hat.

Aufgabe der Erfindung ist es daher, ein Verfahren zum Bestimmen des Gehalts wenigstens eines Analyten in einer Probe der eingangs genannten Art zu schaffen, bei dem eine unvollständige Hydrolyse der Glucuronide des oder der Analyten berücksichtigt wird und das eine exaktere Bestimmung des Gehalts des Analyten in der Probe bei der Probennahme ermöglicht. Ferner ist es Aufgabe der Erfindung, ein verbessertes Analyse-Kit zur Verwendung bei einem solchen Verfahren zu schaffen.

Diese Aufgaben werden erfindungsgemäß durch ein Verfahren mit den Merkmalen des Anspruchs 1 bzw. durch einen Analyse-Kit mit den Merkmalen des Anspruchs 11 gelöst.

Das eingangs genannte Verfahren zum Bestimmen des Gehalts wenigstens eines zumindest teilweise als Glucuronid vorliegenden Analyten in einer Probe, bei dem das wenigstens eine Glucuronid durch Hydrolyse unter Freisetzung des wenigstens einen Analyten aufgespalten wird, der Probe für jeden Analyten eine vorgegebene Menge eines Isotopologs des Analyten als interner Standard zugegeben wird und die Probe nach der Hydrolyse einer quantitativen Analyse zugeführt wird, bei der für jeden Analyten ein Verhältnis des Gehalts des Analyten zu dem Gehalt des zugehörigen Isotopologs bestimmt wird, ist erfindungsgemäß dadurch gekennzeichnet, dass der Probe vor der Aufspaltung durch die Hydrolyse eine vorgegebene Menge wenigstens eines Modell-Glucuronids zugegeben wird. Das eine Modell-Glucuronid oder jedes der mehreren Modell-Glucuronide ist (i) ein Glucuronid eines ersten Isotopologs eines Referenzanalyten, (ii) ein Glucuronid eines ersten Isotopologs der Glucuronsäure und des Referenzanalyten oder (iii) ein erstes Isotopolog des Glucuronids des Referenzanalyten. Ferner wird der Probe vor der quantitativen Analyse eine vorgegebene Menge eines von dem ersten Isotopolog verschiedenen zweiten Isotopologs (i) des Referenzanalyten beziehungsweise (ii) der Glucuronsäure oder (iii) des Glucuronids des Referenzanalyten als interner Standard zugegeben. Schließlich wird bei der quantitativen Analyse das Verhältnis des Gehalts des ersten Isotopologs zu dem Gehalt des zweiten Isotopologs (i) des Referenzanalyten beziehungsweise (ii) der Glucuronsäure oder (iii) des Glucuronids des Referenzanalyten bestimmt. Kerngedanke der Erfindung ist die Zugabe zweier in der quantitativen Analyse qualitativ unterscheidbarer Isotopologe eines Modell-Glucuronids oder eines Bestandteils dieses Modell-Glucuronids, von denen das erste Isotopolog der Hydrolyse unterworfen und der Gehalt des zweiten, als interner Standard zugegebenen Isotopologs von der Hydrolyse nicht beeinflusst wird. Hierbei umfasst die Erfindung drei Grundausführungsformen. Bei einer ersten, bevorzugten Grundausführungsform ist das Modell-Glucuronid ein Glucuronid eines ersten Isotopologs eines Referenzanalyten und wird der Probe vor der quantitativen Analyse eine vorgegebene Menge eines zweiten Isotopologs des Referenzanalyten zugegeben. Bei dieser ersten Grundausführungsform können das Modell-Glucuronid mit dem ersten Isotopolog und das zweite Isotopolog des Referenzanalyten gemeinsam vor der Hydrolyse zugegeben werden. Alternativ kann das zweite Isotopolog des Referenzanalyten auch nach der Hydrolyse zugegeben werden. Bei der zweiten Grundausführungsform ist das Modell-Glucuronid ein Glucuronid eines ersten Isotopologs der Glucuronsäure und des Referenzanalyten und wird der Probe vor der quantitativen Analyse eine vorgegebene Menge eines zweiten Isotopologs der Glucuronsäure zugegeben. Auch bei der zweiten Grundausführungsform kann das zweite Isotopolog der Glucuronsäure vor oder nach der Hydrolyse zugegeben werden. Bei der ersten und der zweiten Grundausführungsform ist das Verhältnis des Gehalts des ersten Isotopologs zu dem Gehalt des zweiten Isotopologs des Referenzanalyten bzw. der Glucuronsäure um so höher, je vollständiger die Hydrolyse ist. Bei einer dritten Grundausführungsform ist das Modell-Glucuronid ein erstes Isotopolog des Glucuronids des Referenzanalyten und wird der Probe vor der quantitativen Analyse eine vorgegebene Menge eines zweiten Isotopologs des Glucuronids des Referenzanalyten zugegeben, wobei in diesem Fall das zweite Isotopolog nur nach der Hydrolyse (und gegebenenfalls nach Entfernung oder Deaktivierung einer dazu verwendeten Glucuronidase) zugegeben werden kann. Bei der dritten Grundausführungsform ist das Verhältnis des Gehalts des ersten Isotopologs zu dem Gehalt des zweiten Isotopologs des Glucuronids des Referenzanalyten um so geringer, je vollständiger die Hydrolyse ist. Bei Ausführungsformen des erfindungsgemäßen Verfahrens, bei denen mehrere Modell-Glucuronide zugegeben werden, sind auch Misch-Ausführungsformen der ersten bis dritten Grundausführungsform denkbar.

Das erfindungsgemäße Verfahren gestattet somit eine Überwachung der Hydrolyse-Effizienz.

Bei einer bevorzugten Ausführungsformen des erfindungsgemäßen Verfahrens wird als Modell-Glucuronid ein Glucuronid verwendet, das im Vergleich zu den Glucuroniden des wenigstens einen Analyten die längste Hydrolysedauer aufweist. Es wird somit vorzugsweise ein Modell-Glucuronid gewählt, welches unter den gegebenen Hydrolysebedingungen schwierig zu hydrolysieren ist. Vorzugsweise wird hier ein isotopen-markiertes Codein-Glucuronid, vorzugsweise ein deuteriertes Codein-Glucuronid, insbesondere Codein-Glucuronid-D₆, verwendet. Dieses eignet sich insbesondere bei Verfahren, bei denen der Gehalt von Drogen analysiert werden soll. Die bevorzugte Ausführungsform des erfindungsgemäßen Verfahrens, bei der ein deuteriertes Codein-Glucuronid als Modell-Glucuronid eingesetzt wird, ist vorzugsweise eine Variante der ersten oben genannten Grundausführungsform des erfindungsgemäßen Verfahrens. Hierbei wird ein zweites Isotopolog des Codeins, insbesondere ein Kohlenstoff- und Stickstoff-Isotope enthaltendes Isotopolog des Codeins, insbesondere Codein-¹³C₄¹⁵N, als interner Standard verwendet. Dabei kann auch der Fall eintreten, dass das Codein selbst einer der in der Patientenprobe zu bestimmenden Analyten ist. In diesem Fall bildet der interne Standard eines der Analyten, nämlich des Codeins, zugleich das zweite Isotopolog des Referenzanalyten.

Grundsätzlich sind beliebige Hydrolyse-Verfahren einsetzbar, um das Aufspalten der Glucuronide zu erreichen. Vorzugsweise jedoch ist das erfindungsgemäße Verfahren dadurch gekennzeichnet, dass eine Glucuronidase, vorzugsweise eine β-Glucuronidase, zugegeben wird. Vorzugsweise wird eine β-Glucuronidase gewählt, die auch bei Codein-Glucuronid eine hohe Hydrolyse-Effizienz zeigt.

Bei einer bevorzugten Weiterbildung des erfindungsgemäßen Verfahrens (in seiner oben genannten ersten oder zweiten Grundausführungsform) werden sowohl das wenigstens eine Isotopolog des Analyten als auch das zweite Isotopolog des Referenzanalyten beziehungsweise das zweite Isotopolog der Glucuronsäure in vorgegebenen Mengenverhältnissen gemischt und wird eine vorgegebene Menge der so hergestellten Isotopologen-Mischung der Probe zugegeben. Bei dieser Ausführungsform wird vorzugsweise das Modell-Glucuronid einem Rekonstitutionspuffer für die Isotopologen-Mischung zugemischt. Dies hat den Vorteil, dass die Zugabe des Modell-Glucuronids als Hydrolyse-Marker automatisch Bestandteil der Probenvorbereitung ist.

Bei einer bevorzugten Ausführungsformen des erfindungsgemäßen Verfahrens umfasst die quantitative Analyse eine Chromatographie, vorzugsweise eine Flüssigkeitschromatographie, besonders bevorzugt eine Hochleistungsflüssigkeitschromatographie, mit einer sich anschließenden Detektionsvorrichtung. Als Detektionsvorrichtung wird vorzugsweise ein Massenspektrometer, besonders bevorzugt ein Tandem-Massenspektrometer, verwendet. Dies ermöglicht eine schnelle sowohl qualitative als auch quantitative Analyse von Proben hinsichtlich einer Vielzahl von Analyten.

Die mit dem erfindungsgemäßen Verfahren zu analysierende Probe basiert üblicherweise auf einer Körperflüssigkeit eines Probanden oder Patienten, bei der die genaue Zusammensetzung der Matrix unbekannt ist. Die Probe kann aber auch eine künstliche Referenzprobe mit bekannter Matrix sein, mit deren Hilfe die Hydrolyse-Effizienz des Hydrolyse-Verfahrensschritts bestimmt werden kann. Bei einer bevorzugten Ausführungsform des erfindungsgemäßen Verfahrens ist die Probe eine Urin-Probe (eines Probanden oder Patienten). Wie eingangs ausgeführt, sind die Analyten, insbesondere zu analysierende Drogen, bei einer Urinprobe häufig teilweise oder sogar überwiegend glucuronidiert.

Eine besonders bevorzugte Weiterbildung des erfindungsgemäßen Verfahrens ist dadurch gekennzeichnet, dass vor der Analyse der Probe die Hydrolyse-Effizienz bestimmt wird, indem zunächst eine Referenzprobe ("Hydrolyse-Kontrolle") mit einer Referenzmatrix und einer vorgegebenen Menge des wenigstens einen Modell-Glucuronids ("Hydrolyse-Marker") bereitgestellt wird. Die Referenzmatrix ist so gewählt, dass Störungen bei der Hydrolyse minimiert werden und eine nahezu vollständige Hydrolyse sichergestellt wird, solange das Enzym eine ausreichende Aktivität zeigt. Dann wird das wenigstens eine Modell-Glucuronid durch Hydrolyse aufgespalten, wobei der wenigstens eine Referenzanalyt freigesetzt wird. Der Referenzprobe wird vor der quantitativen Analyse eine vorgegebene Menge eines von dem ersten Isotopolog verschiedenen zweiten Isotopologs (i) des Referenzanalyten beziehungsweise (ii) der Glucuronsäure oder (iii) des Glucuronids des Referenzanalyten als interner Standard zugegeben. Die Referenzprobe wird nach der Hydrolyse einer quantitativen Analyse zugeführt, bei der das Verhältnis des Gehalts des ersten Isotopologs zu dem Gehalt des zweiten Isotopologs des Referenzanalyten (bei der ersten Grundausführungsform) beziehungsweise der Glucuronsäure (bei der zweiten Grundausführungsform) oder des Glucuronids (bei der dritten Grundausführungsform) bestimmt wird. Dieses Verhältnis stellt die Hydrolyse-Effizienz der Referenzprobe ("Ref-Ratio") dar. Das anschließend bei der Probe bestimmte Verhältnis des Gehalts des ersten Isotopologs zu dem Gehalt des zweiten Isotopologs (i) des Referenzanalyten beziehungsweise (ii) der Glucuronsäure oder (iii) des Glucuronids des Referenzanalyten ist die probenspezifische Hydrolyse-Effizienz ("Sample-Specific Hydrolysis Efficiency Ratio"; "SHE-Ratio"). Schließlich wird ein Verhältnis der probenspezifischen Hydrolyse-Effizienz ("SHE-Ratio") zu der Hydrolyse-Effizienz der Referenzprobe ("Ref-Ratio"), das heißt, des bei der Probe bestimmten Verhältnisses des Gehalts des ersten Isotopologs zu dem Gehalt des zweiten Isotopologs (i) des Referenzanalyten beziehungsweise (ii) der Glucuronsäure oder (iii) des Glucuronids des Referenzanalyten zu dem bei der Referenzprobe bestimmten Verhältnis des Gehalts des ersten Isotopologs zu dem Gehalt des zweiten Isotopologs (i) des Referenzanalyten beziehungsweise (ii) der Glucuronsäure oder (iii) des Glucuronids des Referenzanalyten bestimmt. Dieses wird vorzugsweise normiert dargestellt, d. h. es wird eine auf die Referenzprobe normierte probenspezifische Hydrolyse-Effizienz bestimmt, beispielsweise nach der Formel: ${SHE}_{norm} = \left(\left(SHE-Ratio/Ref-Ratio\right)-1\right) * 100 \left[%\right] .$

Bei dieser bevorzugten Weiterbildung des erfindungsgemäßen Verfahrens wird vorzugsweise die Referenzprobe mit einem oder mehreren weiteren ausgewählten Glucuroniden bereitgestellt.

Das erfindungsgemäße Analyse-Kit zur Verwendung bei einem Verfahren zum Bestimmen des Gehalts wenigstens eines Analyten in einer Probe, in der der wenigstens eine Analyt zumindest teilweise als Glucuronid vorliegt, wobei das Verfahren eine Hydrolyse des wenigstens einen Glucuronids und eine nachfolgende quantitative Analyse des wenigstens einen Analyten einschließt, umfasst eine erste Lösung, die einen Stoff oder eine Stoffmischung, vorzugsweise eine Glucuronidase, zum Aufspalten des wenigstens einen Glucuronids des wenigstens einen Analyten enthält. Die erste Lösung des Analyse-Kits wird beispielsweise als Enzym-Master-Mischung bezeichnet. Das erfindungsgemäße Analyse-Kit umfasst ferner eine zweite Lösung, die eine vorgegebene Menge wenigstens eines Modell-Glucuronids enthält, wobei das Modell-Glucuronid (i) - in einer ersten Grundausführungsform - ein Glucuronid eines ersten Isotopologs eines Referenzanalyten (ii) - in einer zweiten Grundausführungsform - ein Glucuronid eines ersten Isotopologs der Glucuronsäure und des Referenzanalyten oder (iii) - in einer dritten Grundausführungsform - ein erstes Isotopolog des Glucuronids des Referenzanalyten ist. Die zweite Lösung des Analyse-Kits wird beispielsweise als Hydrolyse-Marker bezeichnet. Das erfindungsgemäße Analyse-Kit umfasst schließlich eine Isotopologen-Mischung, die für jeden Analyten eine vorgegebene Menge eines Isotopologs als internen Standard enthält, wobei die Isotopologen-Mischung (i) - in der ersten Grundausführungsform - eine vorgegebene Menge eines von dem ersten Isotopolog verschiedenen zweiten Isotopologs des Referenzanalyten beziehungsweise (ii) - in der zweiten Grundausführungsform - eine vorgegebene Menge eines von dem ersten Isotopolog verschiedenen zweiten Isotopologs der Glucuronsäure oder (iii) - in der dritten Grundausführungsform - eine vorgegebene Menge eines von dem ersten Isotopolog verschiedenen zweiten Isotopologs des Glucuronids des Referenzanalyten enthält. Die Isotopologen-Mischung des Analyse-Kits wird auch als Interne-Standards-Mischung bezeichnet. Bei der ersten oder der zweiten Grundausführungsform können die zweite Lösung (Hydrolyse-Marker) und die Isotopologen-Mischung des Analyse-Kits auch zu einer Mischung vereint sein.

Das Modell-Glucuronid ist vorzugsweise ein Glucuronid, das im Vergleich zu den Glucuroniden des wenigstens einen Analyten die längste Hydrolysedauer aufweist. Als Modell-Glucuronid besonders bevorzugt ist ein isotopen-markiertes Codein-Glucuronid, vorzugsweise ein deuteriertes Codein-Glucuronid, insbesondere Codein-Glucuronid-D₆. Bei dieser Ausführungsform ist das zweite Isotopolog des Referenzanalyten ein ¹³C¹⁵N-markiertes Codein, vorzugsweise Codein-¹³C₄¹⁵N, beziehungsweise das zweite Isotopolog der Glucuronsäure eine ¹³C¹⁵N-markierte Glucuronsäure.

Bei einer Weiterbildung des erfindungsgemäßen Analyse-Kits (der ersten oder der zweiten Grundausführungsform) ist die Lösung, die die vorgegebene Menge des Modell-Glucuronids enthält, ein Rekonstitutionspuffer für die Isotopologen-Mischung.

Eine bevorzugte Ausführungsform des Analyse-Kits zur Verwendung in einem Verfahren zum Bestimmen des Gehalts wenigstens eines Analyten in einer Probe, bei dem vor der Analyse der Probe die Hydrolyse-Effizienz bestimmt wird, ist durch eine Referenzprobe mit einer Referenzmatrix, einer vorgegebenen Menge des wenigstens einen Modell-Glucuronids und einem oder mehreren weiteren ausgewählten Glucuroniden gekennzeichnet.

Vorteilhafte und/oder bevorzugte Ausführungsformen der Erfindung sind in den Unteransprüchen gekennzeichnet.

Nachfolgend wird die Erfindung anhand von in der Zeichnung dargestellten bevorzugten Ausführungsbeispielen näher beschrieben. In der Zeichnung zeigen:
Figur 1 eine schematische Darstellung des Verfahrensablaufs einer bevorzugten Ausführungsform des erfindungsgemäßen Verfahrens und
Figur 2 eine schematische Darstellung der sich bei der Hydrolyse ergebenden Änderungen der Konzentrationen der Isotopologe des Codeins.

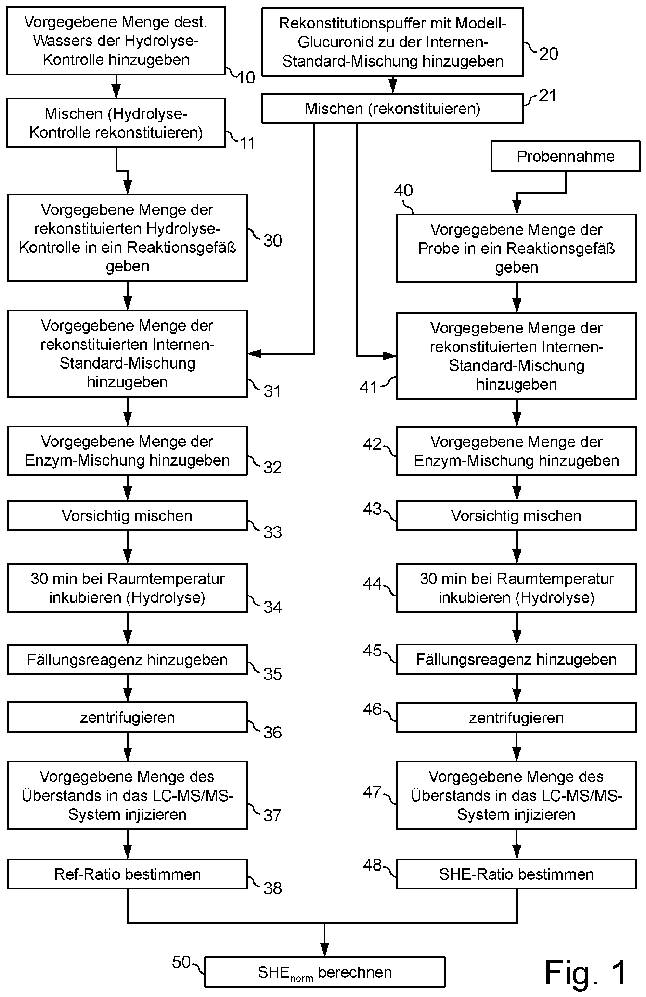

Eine bevorzugte Ausführungsform des erfindungsgemäßen Verfahrens zum Bestimmen des Gehalts wenigstens eines Analyten in einer Probe wird anhand der Figur 1 beschrieben. Zur Vereinfachung werden hier nicht sämtliche Verfahrensschritte beschrieben, die der eigentlichen Analyse vorausgehen, wie beispielsweise das Kalibrieren oder die Analyse von Kontroll-Proben. Die anhand von Figur 1 beschriebene Ausführungsform umfasst die Überprüfung der Hydrolyse-Effizienz unter Verwendung einer Hydrolyse-Kontrollprobe (kurz als "Hydrolyse-Kontrolle" bezeichnet) sowie die eigentliche Analyse der einem Probanden oder Patienten entnommenen Probe.

In einem ersten vorbereitenden Schritt 10 wird einer in einem Fläschchen eines Analyse-Kits bereitgestellten Hydrolyse-Kontrollprobe, die eine Urin-Matrix und eine Auswahl von Drogen in glucuronidierter Form in vorgegebenen Mengen enthält, eine vorgegebene Menge destillierten Wassers zugegeben (mittels Pipettieren). Zum Rekonstituieren wird die Mischung für eine vorgegebene Zeitdauer bei einer vorgegebenen Temperatur durch wiederholtes Rühren gemischt (Schritt 11). Die so erzeugte rekonstituierte Hydrolyse-Kontrolle kann dann für wenige Tage gelagert werden und wird für die nachfolgend beschriebenen Verfahrensschritte bereitgestellt.

In einem weiteren vorbereitenden Schritt 20 wird eine vorgegebene Menge eines in dem Analyse-Kit bereitgestellten Rekonstitutionspuffers, der unter anderem ein Modell-Glucuronid enthält, einer in einem weiteren Fläschchen des Analyse-Kits bereitgestellten Mischung interner Standards zugegeben. Wie dies auch in der eingangs genannten Broschüre "MassTox^{®} - Drogenanalytik im Urin mit LC-MS/MS" beschrieben ist, enthält die Interne-Standards-Mischung eine Mehrzahl interner Standards, beispielsweise isotopen-markierte interne Standards für etwa 100 Analyten. Das Modell-Glucuronid des Rekonstitutionspuffers ist ein Glucuronid eines ersten Isotopologs des Referenzanalyten Codein, wobei vorzugsweise ein deuteriertes Codein-Glucuronid-D₆ enthalten ist. Zum Rekonstituieren wird die Mischung für eine vorgegebene Zeitdauer bei einer vorgegebenen Temperatur durch wiederholtes Rühren gemischt (Schritt 21). Die so erzeugte rekonstituierte Interne-Standard-Mischung kann dann für eine vorgegebene Zeitdauer unter vorgegebenen Temperaturbedingungen gelagert werden und wird für die nachfolgend beschriebenen Verfahrensschritte bereitgestellt.

Die Hydrolyse-Kontrollprobe wird dann demselben Verfahren zum Bestimmen des Gehalts der Analyten (Drogen) unterzogen, dem anschließend auch die einem Probanden oder Patienten entnommene Urinprobe unterzogen werden soll. Dazu wird zunächst eine vorgegebene Menge der Hydrolyse-Kontrollprobe (beispielsweise 50 µl) in ein bereitgestelltes Reaktionsgefäß gegeben (Schritt 30). Dann wird eine vorgegebene Menge (beispielsweise 10 µl) der im Schritt 21 vorbereiteten rekonstituierten Interner-Standard-Mischung zugegeben (Schritt 31). Ferner wird eine vorgegebene Menge (beispielsweise 300 µl) einer Enzym-Mischung hinzugefügt (Schritt 32), wobei die Reihenfolge der Schritte 31 und 32 auch abweichen kann. Diese Komponenten werden vorsichtig gemischt (Schritt 33). Anschließend wird die Mischung für eine vorgegebene Zeitdauer (beispielsweise 30 Minuten) bei einer vorgegebenen Temperatur (beispielsweise Raumtemperatur) inkubiert (Schritt 34), wobei die Hydrolyse der Glucuronide, das heißt deren Aufspaltung in die Drogen (Analyten) und Glucuronsäure, stattfindet.

Anschließend wird der Mischung eine Fällungsreagenz zugegeben (Schritt 35), die ebenfalls Bestandteil des Analyse-Kits ist. Dabei werden unter anderem die Enzyme ausgefällt. Nach dem Ausfällen wird die Mischung zur Abtrennung der ausgefällten Stoffe zentrifugiert (Schritt 36). Anschließend wird eine vorgegebene Menge des Überstands entnommen und in das LC-MS/MS-System injiziert.

Es schließt sich die quantitative Analyse mittels HPLC-MS/MS an, wobei unter anderem das die Hydrolyse-Effizienz der Hydrolyse-Kontrollprobe darstellende Referenzverhältnis "Ref-Ratio", das heißt, das Verhältnis des Gehalts des ersten Isotopologs des Referenzanalyten, dem aus dem Codein-Glucuronid abgespaltenen Codein-D₆, zu dem Gehalt des zweiten Isotopologs des Referenzanalyten, dem in der Interne-Standard-Mischung enthaltenen Codein-¹³C₄¹⁵N, bestimmt wird (Schritt 38).

Nach der Analyse der Hydrolyse-Kontrollprobe oder auch teilweise parallel zu dieser wird die dem Probanden oder Patienten entnommene Probe demselben Verfahren unterzogen. Dazu wird zunächst eine vorgegebene Menge der Probe (beispielsweise 50 µl) in ein bereitgestelltes Reaktionsgefäß gegeben (Schritt 40). Dann wird eine vorgegebene Menge (beispielsweise 10 µl) der im Schritt 21 vorbereiteten rekonstituierten Interner-Standard-Mischung zugegeben (Schritt 41). Ferner wird eine vorgegebene Menge (beispielsweise 300 µl) einer Enzym-Mischung hinzugefügt (Schritt 42), wobei die Reihenfolge der Schritte 41 und 42 auch abweichen kann. Diese Komponenten werden vorsichtig gemischt (Schritt 43). Anschließend wird die Mischung für eine vorgegebene Zeitdauer (beispielsweise 30 Minuten) bei einer vorgegebenen Temperatur (beispielsweise Raumtemperatur) inkubiert (Schritt 44), wobei die Hydrolyse der Glucuronide, das heißt deren Aufspaltung in die Drogen (Analyten) und Glucuronsäure, stattfindet.

Anschließend wird der Mischung die Fällungsreagenz zugegeben (Schritt 45). Nach dem Ausfällen wird die Mischung zur Abtrennung der ausgefällten Stoffe zentrifugiert (Schritt 46). Anschließend wird eine vorgegebene Menge des Überstands entnommen und in das LC-MS/MS-System injiziert.

Es schließt sich die quantitative Analyse mittels HPLC-MS/MS an, wobei unter anderem das die probenspezifische Hydrolyse-Effizienz darstellende Verhältnis "SHE-Ratio", das heißt, das Verhältnis des Gehalts des ersten Isotopologs des Referenzanalyten, dem aus dem Codein-Glucuronid abgespaltenen Codein-D₆, zu dem Gehalt des zweiten Isotopologs des Referenzanalyten, dem in der Interne-Standard-Mischung enthaltenen Codein-¹³C₄¹⁵N, bestimmt wird (Schritt 48).

Im Schritt 50 wird dann die probenspezifische Hydrolyse-Effizienz SHE-Ratio auf die Hydrolyse-Effizienz der Hydrolyse-Kontrolle Betreff-Ratio normiert nach der Formel: ${SHE}_{norm} = \left(\left(SHE-Ratio/Ref-Ratio\right)-1\right) * 100 \left[%\right] .$

Auf der Grundlage des Werts SHEₙₒᵣₘ wird die "schwierigste" Hydrolyse, nämlich die des Codein-Glucuronids, bewertet. Bei Werten von SHEₙₒᵣₘ > -50 % kann beispielsweise davon ausgegangen werden, dass die Hydrolyse anderer Stoffe, wie beispielsweise Temazepam-Glucuronid, Oxazepam-Glucuronid und 11-Nor-9-Carboxy-Δ9-THC-Glucuronid bereits vollständig abgeschlossen ist.

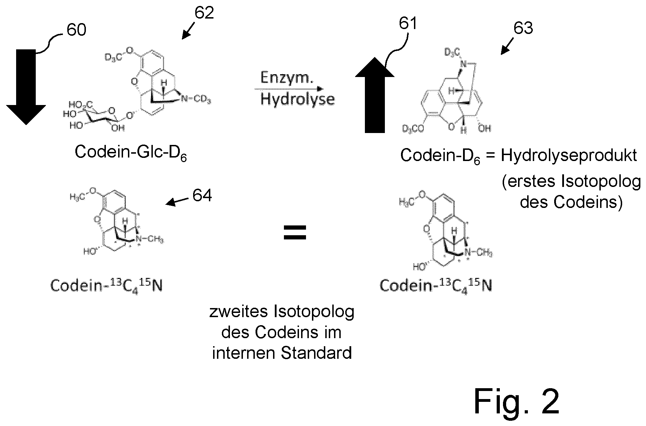

Figur 2 veranschaulicht die sich bei der Hydrolyse ergebenden Änderungen der Konzentrationen der Isotopologe des Codeins. Vor der Hydrolyse (Schritte 34 bzw. 44 gemäß Figur 1) befinden sich in der Mischung das über den Rekonstitutionspuffer zugegebene Codein-Glucuronid-D₆ 62 und das über die Interne-Standard-Mischung zugegebene Codein-¹³C₄¹⁵N 64, wie es auf der linken Seite der Figur 2 dargestellt ist. Während der Hydrolyse sinkt die Konzentration des Codein-Glucuronids-D₆ 62 und steigt die Konzentration des Hydrolyseprodukts Codein-D₆ 63, wobei die Konzentrationsänderungen durch die Pfeile 60 bzw. 61 veranschaulicht sind. Je größer im Ergebnis das Verhältnis der Konzentration des Codein-D₆ 63 zu der des Codein-¹³C₄¹⁵N 64 ist, desto effizienter war die Hydrolyse.

Im Rahmen des Erfindungsgedankens sind zahlreiche alternativer Ausführungsformen denkbar. Sämtlichen Ausführungsformen ist gemeinsam, dass einer Probe, in der die Analyten zumindest teilweise in glucuronidierter Form vorliegen, zwei in der quantitativen Analyse qualitativ unterscheidbare Isotopologe eines Bestandteils eines Modell-Glucuronids oder des Modell-Glucuronids selbst zugegeben werden, von denen das erste Isotopolog der Hydrolyse unterworfen und der Gehalt des zweiten, als interner Standard zugegebenen Isotopologs von der Hydrolyse nicht beeinflusst wird.

## Patentansprüche

1. Verfahren zum Bestimmen des Gehalts wenigstens eines Analyten in einer Probe, wobei der wenigstens eine Analyt zumindest teilweise als Glucuronid vorliegt,
wobei das wenigstens eine Glucuronid durch Hydrolyse unter Freisetzung des wenigstens einen Analyten aufgespalten wird,
wobei der Probe für jeden Analyten eine vorgegebene Menge eines Isotopologs des Analyten als interner Standard zugegeben wird,
wobei die Probe nach der Hydrolyse einer quantitativen Analyse zugeführt wird, bei der für jeden Analyten ein Verhältnis des Gehalts des Analyten zu dem Gehalt des zugehörigen Isotopologs bestimmt wird,
**dadurch gekennzeichnet,**
**dass** der Probe vor der Aufspaltung durch die Hydrolyse eine vorgegebene Menge wenigstens eines Modell-Glucuronids zugegeben wird, wobei das Modell-Glucuronid (i) ein Glucuronid eines ersten Isotopologs eines Referenzanalyten, (ii) ein Glucuronid eines ersten Isotopologs der Glucuronsäure und des Referenzanalyten oder (iii) ein erstes Isotopolog des Glucuronids des Referenzanalyten ist,
**dass** der Probe vor der quantitativen Analyse eine vorgegebene Menge eines von dem ersten Isotopolog verschiedenen zweiten Isotopologs (i) des Referenzanalyten beziehungsweise (ii) der Glucuronsäure oder (iii) des Glucuronids des Referenzanalyten als interner Standard zugegeben wird, und
**dass** bei der quantitativen Analyse ferner das Verhältnis des Gehalts des ersten Isotopologs zu dem Gehalt des zweiten Isotopologs (i) des Referenzanalyten beziehungsweise (ii) der Glucuronsäure oder (iii) des Glucuronids des Referenzanalyten bestimmt wird.

2. Verfahren nach Anspruch 1, **dadurch gekennzeichnet, dass** als Modell-Glucuronid ein Glucuronid verwendet wird, das im Vergleich zu den Glucuroniden des wenigstens einen Analyten die längste Hydrolysedauer aufweist.

3. Verfahren nach einem der Ansprüche 1 - 2, **dadurch gekennzeichnet, dass** das wenigstens eine Glucuronid durch Hydrolyse aufgespalten wird, indem eine Glucuronidase, vorzugsweise eine β-Glucuronidase, zugegeben wird.

4. Verfahren nach einem der Ansprüche 1 - 3, **dadurch gekennzeichnet, dass** sowohl das wenigstens eine Isotopolog des Analyten als auch das zweite Isotopolog des Referenzanalyten beziehungsweise das zweite Isotopolog der Glucuronsäure in vorgegebenen Mengenverhältnissen gemischt werden und eine vorgegebene Menge der so hergestellten Isotopologen-Mischung der Probe zugegeben wird.

5. Verfahren nach Anspruch 4, **dadurch gekennzeichnet, dass** das Modell-Glucuronid einem Rekonstitutionspuffer für die Isotopologen-Mischung zugemischt wird.

6. Verfahren nach einem der Ansprüche 1 - 5, **dadurch gekennzeichnet, dass** die quantitative Analyse eine Chromatographie, vorzugsweise eine Hochleistungsflüssigkeitschromatographie, mit einer Detektionsvorrichtung umfasst.

7. Verfahren nach Anspruch 6, **dadurch gekennzeichnet, dass** als Detektionsvorrichtung ein Massenspektrometer, vorzugsweise ein Tandem-Massenspektrometer, verwendet wird.

8. Verfahren nach einem der Ansprüche 1 - 7, **dadurch gekennzeichnet, dass** die Probe eine Urin-Probe ist.

9. Verfahren zum Bestimmen des Gehalts wenigstens eines Analyten in einer Probe nach einem der Ansprüche 1 - 8, **dadurch gekennzeichnet,**
**dass** vor der Analyse der Probe die Hydrolyse-Effizienz bestimmt wird, indem:
eine Referenzprobe mit einer Referenzmatrix und einer vorgegebenen Menge des wenigstens einen Modell-Glucuronids bereitgestellt wird,
das wenigstens eine Modell-Glucuronid durch Hydrolyse aufgespalten werden, wobei der wenigstens eine Referenzanalyt freigesetzt wird,
wobei der Referenzprobe vor der quantitativen Analyse eine vorgegebene Menge eines von dem ersten Isotopolog verschiedenen zweiten Isotopologs (i) des Referenzanalyten beziehungsweise (ii) der Glucuronsäure oder (iii) des Glucuronids des Referenzanalyten als interner Standard zugegeben wird,
wobei die Referenzprobe nach der Hydrolyse einer quantitativen Analyse zugeführt wird, bei der das Verhältnis des Gehalts des ersten Isotopologs zu dem Gehalt des zweiten Isotopologs des Referenzanalyten beziehungsweise der Glucuronsäure bestimmt wird, und
**dass** ein Verhältnis des bei der Probe bestimmten Verhältnisses des Gehalts des ersten Isotopologs zu dem Gehalt des zweiten Isotopologs (i) des Referenzanalyten beziehungsweise (ii) der Glucuronsäure oder (iii) des Glucuronids des Referenzanalyten zu dem bei der Referenzprobe bestimmten Verhältnis des Gehalts des ersten Isotopologs zu dem Gehalt des zweiten Isotopologs (i) des Referenzanalyten beziehungsweise (ii) der Glucuronsäure oder (iii) des Glucuronids des Referenzanalyten bestimmt wird.

10. Verfahren nach Anspruch 9, **dadurch gekennzeichnet, dass** die Referenzprobe mit einem oder mehreren weiteren ausgewählten Glucuroniden bereitgestellt wird.

11. Analyse-Kit zur Verwendung bei einem Verfahren zum Bestimmen des Gehalts wenigstens eines Analyten in einer Probe, in der der wenigstens eine Analyt zumindest teilweise als Glucuronid vorliegt, wobei das Verfahren eine Hydrolyse des wenigstens einen Glucuronids und eine nachfolgende quantitative Analyse des wenigstens einen Analyten umfasst, wobei das Kit aufweist:
eine erste Lösung, die einen Stoff oder eine Stoffmischung, vorzugsweise eine Glucuronidase, zum Aufspalten des wenigstens einen Glucuronids des wenigstens einen Analyten enthält,
eine zweite Lösung, die eine vorgegebene Menge wenigstens eines Modell-Glucuronids enthält, wobei das Modell-Glucuronid (i) ein Glucuronid eines ersten Isotopologs eines Referenzanalyten (ii) ein Glucuronid eines ersten Isotopologs der Glucuronsäure und des Referenzanalyten oder (iii) ein erstes Isotopolog des Glucuronids des Referenzanalyten ist, und
eine Isotopologen-Mischung, die für jeden Analyten eine vorgegebene Menge eines Isotopologs als internen Standard enthält,
wobei die Isotopologen-Mischung (i) eine vorgegebene Menge eines von dem ersten Isotopolog verschiedenen zweiten Isotopologs des Referenzanalyten beziehungsweise (ii) eine vorgegebene Menge eines von dem ersten Isotopolog verschiedenen zweiten Isotopologs der Glucuronsäure oder (iii) eine vorgegebene Menge eines von dem ersten Isotopolog verschiedenen zweiten Isotopologs des Glucuronids des Referenzanalyten enthält.

12. Analyse-Kit nach Anspruch 11, **dadurch gekennzeichnet, dass** das Modell-Glucuronid ein Glucuronid ist, das im Vergleich zu den Glucuroniden des wenigstens einen Analyten die längste Hydrolysedauer aufweist.

13. Analyse-Kit nach Anspruch 12, **dadurch gekennzeichnet, dass** das Modell-Glucuronid ein isotopen-markiertes Codein-Glucuronid, vorzugsweise ein deuteriertes Codein-Glucuronid, insbesondere Codein-Glucuronid-D₆, ist.

14. Analyse-Kit nach Anspruch 13, **dadurch gekennzeichnet, dass** das zweite Isotopolog des Referenzanalyten ein ¹³C¹⁵N-markiertes Codein, vorzugsweise Codein-¹³C₄¹⁵N, ist beziehungsweise das zweite Isotopolog der Glucuronsäure eine ¹³C¹⁵N-markierte Glucuronsäure ist.

15. Analyse-Kit nach einem der Ansprüche 11 - 14, **dadurch gekennzeichnet, dass** die Lösung, die die vorgegebene Menge des Modell-Glucuronids enthält, ein Rekonstitutionspuffer für die Isotopologen-Mischung ist.

16. Analyse-Kit nach einem der Ansprüche 11 - 15 zur Verwendung in einem Verfahren zum Bestimmen des Gehalts wenigstens eines Analyten in einer Probe, bei dem vor der Analyse der Probe die Hydrolyse-Effizienz bestimmt wird, **gekennzeichnet durch** eine Referenzprobe mit einer Referenzmatrix, einer vorgegebenen Menge des wenigstens einen Modell-Glucuronids und einem oder mehreren weiteren ausgewählten Glucuroniden.

## Claims

1. Method for determining the content of at least one analyte in a sample, wherein the at least one analyte is present at least partially as a glucuronide,
wherein the at least one glucuronide is cleaved by hydrolysis with release of the at least one analyte,
wherein a predetermined amount of an isotopologue of the analyte is added to the sample, for each analyte, as an internal standard,
wherein the sample, after the hydrolysis, is subjected to a quantitative analysis in which, for each analyte, a ratio of the content of the analyte to the content of the associated isotopologue is determined,
**characterised in that**
a predetermined amount of at least one model glucuronide is added to the sample before cleavage by hydrolysis, wherein the model glucuronide is (i) a glucuronide of a first isotopologue of a reference analyte, (ii) a glucuronide of a first isotopologue of glucuronic acid and of the reference analyte, or (iii) a first isotopologue of the glucuronide of the reference analyte,
a predetermined amount of a second isotopologue, different from the first isotopologue, (i) of the reference analyte, (ii) of the glucuronic acid or (iii) of the glucuronide of the reference analyte, respectively, is added to the sample before the quantitative analysis as an internal standard, and
in the quantitative analysis, a ratio of the content of the first isotopologue to the content of the second isotopologue (i) of the reference analyte, (ii) of the glucuronic acid or (iii) of the glucuronide of the reference analyte, respectively, is further determined.

2. Method according to claim 1, **characterised in that** the model glucuronide used is a glucuronide which, in comparison with the glucuronides of the at least one analyte, has the longest hydrolysis time.

3. Method according to any one of claims 1 to 2, **characterised in that** the at least one glucuronide is cleaved by hydrolysis by adding a glucuronidase, preferably a β-glucuronidase.

4. Method according to any one of claims 1 to 3, **characterised in that** both the at least one isotopologue of the analyte and the second isotopologue of the reference analyte or the second isotopologue of the glucuronic acid, respectively, are mixed in predetermined quantitative ratios, and a predetermined amount of the isotopologue mixture thus prepared is added to the sample.

5. Method according to claim 4, **characterised in that** the model glucuronide is added to a reconstitution buffer for the isotopologue mixture.

6. Method according to any one of claims 1 to 5, **characterised in that** the quantitative analysis comprises chromatography, preferably high-performance liquid chromatography, with a detection device.

7. Method according to claim 6, **characterised in that** the detection device used is a mass spectrometer, preferably a tandem mass spectrometer.

8. Method according to any one of claims 1 to 7, **characterised in that** the sample is a urine sample.

9. Method for determining the content of at least one analyte in a sample according to any one of claims 1 to 8, **characterised**
**in that** the hydrolysis efficiency is determined, before analysis of the sample, by:
providing a reference sample with a reference matrix and a predetermined amount of the at least one model glucuronide,
cleaving the at least one model glucuronide by hydrolysis, wherein the at least one reference analyte is released,
adding, to the reference sample before the quantitative analysis, a predetermined amount of a second isotopologue, different from the first isotopologue, (i) of the reference analyte, (ii) of the glucuronic acid, or (iii) of the glucuronide of the reference analyte, respectively, as an internal standard,
subjecting the reference sample, after the hydrolysis, to a quantitative analysis in which the ratio of the content of the first isotopologue to the content of the second isotopologue of the reference analyte, or, as applicable, of the glucuronic acid, is determined, and
**in that** a ratio between the ratio, determined for the sample, of the content of the first isotopologue to the content of the second isotopologue (i) of the reference analyte, (ii) of the glucuronic acid, or (iii) of the glucuronide of the reference analyte, respectively, and the ratio, determined for the reference sample, of the content of the first isotopologue to the content of the second isotopologue (i) of the reference analyte, (ii) of the glucuronic acid, or (iii) of the glucuronide of the reference analyte, respectively, is determined.

10. Method according to claim 9, **characterised in that** the reference sample is provided with one or more further selected glucuronides.

11. Analysis kit for use in a method for determining the content of at least one analyte in a sample, in which the at least one analyte is present at least partially as a glucuronide, wherein the method comprises a hydrolysis of the at least one glucuronide and a subsequent quantitative analysis of the at least one analyte, wherein the kit comprises:
a first solution containing a substance or a mixture of substances, preferably a glucuronidase, for cleaving the at least one glucuronide of the at least one analyte,
a second solution containing a predetermined amount of at least one model glucuronide, wherein the model glucuronide is (i) a glucuronide of a first isotopologue of a reference analyte, (ii) a glucuronide of a first isotopologue of glucuronic acid and of the reference analyte, or (iii) a first isotopologue of the glucuronide of the reference analyte, and
an isotopologue mixture containing, for each analyte, a predetermined amount of an isotopologue as an internal standard,
wherein the isotopologue mixture contains (i) a predetermined amount of a second isotopologue, different from the first isotopologue, of the reference analyte, (ii) a predetermined amount of a second isotopologue, different from the first isotopologue, of the glucuronic acid, or (iii) a predetermined amount of a second isotopologue, different from the first isotopologue, of the glucuronide of the reference analyte, respectively.

12. Analysis kit according to claim 11, **characterised in that** the model glucuronide is a glucuronide which, in comparison with the glucuronides of the at least one analyte, has the longest hydrolysis time.

13. Analysis kit according to claim 12, **characterised in that** the model glucuronide is an isotopically labelled codeine glucuronide, preferably a deuterated codeine glucuronide, in particular codeine glucuronide-D₆.

14. Analysis kit according to claim 13, **characterised in that** the second isotopologue of the reference analyte is ¹³C¹⁵N-labelled codeine, preferably codeine-¹³C₄¹⁵N, or, as applicable, the second isotopologue of the glucuronic acid is ¹³C¹⁵N-labelled glucuronic acid.

15. Analysis kit according to any one of claims 11 to 14, **characterised in that** the solution containing the predetermined amount of the model glucuronide is a reconstitution buffer for the isotopologue mixture.

16. Analysis kit according to any one of claims 11 to 15 for use in a method for determining the content of at least one analyte in a sample, in which the hydrolysis efficiency is determined before analysis of the sample, **characterised in that** the kit comprises a reference sample comprising a reference matrix, a predetermined amount of the at least one model glucuronide, and one or more further selected glucuronides.

## Revendications

1. Procédé de détermination de la teneur d'au moins un analyte dans un échantillon, ledit au moins un analyte étant présent au moins partiellement sous forme de glucuronide,
dans lequel ledit au moins un glucuronide est clivé par hydrolyse avec libération dudit au moins un analyte,
dans lequel une quantité prédéterminée d'un isotopologue de l'analyte est ajoutée à l'échantillon, pour chaque analyte, en tant qu'étalon interne,
dans lequel l'échantillon, après l'hydrolyse, est soumis à une analyse quantitative dans laquelle, pour chaque analyte, un rapport de la teneur de l'analyte à la teneur de l'isotopologue associé est déterminé,
**caractérisé**
**en ce qu'**une quantité prédéterminée d'au moins un glucuronide modèle est ajoutée à l'échantillon avant le clivage par hydrolyse, ledit glucuronide modèle étant (i) un glucuronide d'un premier isotopologue d'un analyte de référence, (ii) un glucuronide d'un premier isotopologue d'acide glucuronique et dudit analyte de référence, ou (iii) un premier isotopologue du glucuronide dudit analyte de référence,
**en ce qu'**une quantité prédéterminée d'un second isotopologue, différent du premier isotopologue, (i) dudit analyte de référence ou, le cas échéant, (ii) dudit acide glucuronique, ou (iii) du glucuronide dudit analyte de référence, est ajoutée à l'échantillon avant l'analyse quantitative en tant qu'étalon interne, et
**en ce que**, lors de l'analyse quantitative, un rapport de la teneur du premier isotopologue à la teneur du second isotopologue (i) dudit analyte de référence ou, le cas échéant, (ii) dudit acide glucuronique, ou (iii) du glucuronide dudit analyte de référence, est en outre déterminé.

2. Procédé selon la revendication 1, **caractérisé en ce que** le glucuronide modèle utilisé est un glucuronide qui présente, par comparaison avec les glucuronides dudit au moins un analyte, la durée d'hydrolyse la plus longue.

3. Procédé selon l'une quelconque des revendications 1 à 2, **caractérisé en ce que** ledit au moins un glucuronide est clivé par hydrolyse par addition d'une glucuronidase, de préférence d'une β-glucuronidase.

4. Procédé selon l'une quelconque des revendications 1 à 3, **caractérisé en ce que** ledit au moins un isotopologue de l'analyte ainsi que le second isotopologue de l'analyte de référence ou, le cas échéant, le second isotopologue de l'acide glucuronique sont mélangés dans des rapports quantitatifs prédéterminés, et **en ce qu'**une quantité prédéterminée du mélange d'isotopologues ainsi préparé est ajoutée à l'échantillon.

5. Procédé selon la revendication 4, **caractérisé en ce que** le glucuronide modèle est ajouté à un tampon de reconstitution destiné au mélange d'isotopologues.

6. Procédé selon l'une quelconque des revendications 1 à 5, **caractérisé en ce que** l'analyse quantitative comprend une chromatographie, de préférence une chromatographie liquide à haute performance, avec un dispositif de détection.

7. Procédé selon la revendication 6, **caractérisé en ce que** le dispositif de détection utilisé est un spectromètre de masse, de préférence un spectromètre de masse en tandem.

8. Procédé selon l'une quelconque des revendications 1 à 7, **caractérisé en ce que** l'échantillon est un échantillon d'urine.

9. Procédé de détermination de la teneur d'au moins un analyte dans un échantillon selon l'une quelconque des revendications 1 à 8, **caractérisé**
**en ce que** l'efficacité d'hydrolyse est déterminée, avant l'analyse de l'échantillon, par les étapes consistant à:
fournir un échantillon de référence comportant une matrice de référence et une quantité prédéterminée dudit au moins un glucuronide modèle,
cliver ledit au moins un glucuronide modèle par hydrolyse, ledit au moins un analyte de référence étant libéré,
ajouter à l'échantillon de référence, avant l'analyse quantitative, une quantité prédéterminée d'un second isotopologue, différent du premier isotopologue, (i) dudit analyte de référence ou, le cas échéant, (ii) dudit acide glucuronique, ou (iii) du glucuronide dudit analyte de référence, en tant qu'étalon interne,
soumettre l'échantillon de référence, après l'hydrolyse, à une analyse quantitative dans laquelle le rapport de la teneur du premier isotopologue à la teneur du second isotopologue dudit analyte de référence ou, le cas échéant, dudit acide glucuronique est déterminé,
**en ce qu'**un rapport entre le rapport, déterminé pour l'échantillon, de la teneur du premier isotopologue à la teneur du second isotopologue (i) dudit analyte de référence ou, le cas échéant, (ii) dudit acide glucuronique, ou (iii) du glucuronide dudit analyte de référence, et le rapport, déterminé pour l'échantillon de référence, de la teneur du premier isotopologue à la teneur du second isotopologue (i) dudit analyte de référence ou, le cas échéant, (ii) dudit acide glucuronique, ou (iii) du glucuronide dudit analyte de référence, est déterminé.

10. Procédé selon la revendication 9, **caractérisé en ce que** l'échantillon de référence est fourni avec un ou plusieurs autres glucuronides sélectionnés.

11. Kit d'analyse destiné à être utilisé dans un procédé de détermination de la teneur d'au moins un analyte dans un échantillon, dans lequel ledit au moins un analyte est présent au moins partiellement sous forme de glucuronide, le procédé comprenant une hydrolyse dudit au moins un glucuronide et une analyse quantitative ultérieure dudit au moins un analyte, le kit comprenant:
une première solution contenant une substance ou un mélange de substances, de préférence une glucuronidase, destiné au clivage dudit au moins un glucuronide dudit au moins un analyte,
une deuxième solution contenant une quantité prédéterminée d'au moins un glucuronide modèle, ledit glucuronide modèle étant (i) un glucuronide d'un premier isotopologue d'un analyte de référence, (ii) un glucuronide d'un premier isotopologue de l'acide glucuronique et dudit analyte de référence, ou (iii) un premier isotopologue du glucuronide dudit analyte de référence, et
un mélange d'isotopologues contenant, pour chaque analyte, une quantité prédéterminée d'un isotopologue en tant qu'étalon interne,
ledit mélange d'isotopologues contenant (i) une quantité prédéterminée d'un second isotopologue, différent du premier isotopologue, dudit analyte de référence ou, le cas échéant, (ii) une quantité prédéterminée d'un second isotopologue, différent du premier isotopologue, dudit acide glucuronique, ou (iii) une quantité prédéterminée d'un second isotopologue, différent du premier isotopologue, du glucuronide dudit analyte de référence.

12. Kit d'analyse selon la revendication 11, **caractérisé en ce que** le glucuronide modèle est un glucuronide qui présente, par comparaison avec les glucuronides dudit au moins un analyte, la durée d'hydrolyse la plus longue.

13. Kit d'analyse selon la revendication 12, **caractérisé en ce que** le glucuronide modèle est un glucuronide de codéine marqué isotopiquement, de préférence un glucuronide de codéine deutéré, en particulier le glucuronide de codéine-D₆.

14. Kit d'analyse selon la revendication 13, **caractérisé en ce que** le second isotopologue de l'analyte de référence est de la codéine marquée au ¹³C¹⁵N, de préférence de la codéine¹³C₄¹⁵N, ou, le cas échéant, **en ce que** le second isotopologue de l'acide glucuronique est de l'acide glucuronique marqué au ¹³C¹⁵N.

15. Kit d'analyse selon l'une quelconque des revendications 11 à 14, **caractérisé en ce que** la solution contenant la quantité prédéterminée du glucuronide modèle est un tampon de reconstitution destiné au mélange d'isotopologues.

16. Kit d'analyse selon l'une quelconque des revendications 11 à 15, destiné à être utilisé dans un procédé de détermination de la teneur d'au moins un analyte dans un échantillon, dans lequel l'efficacité d'hydrolyse est déterminée avant l'analyse de l'échantillon, **caractérisé en ce que** le kit comprend un échantillon de référence comprenant une matrice de référence, une quantité prédéterminée dudit au moins un glucuronide modèle, et un ou plusieurs autres glucuronides sélectionnés.
